# EUROPEAN PATENT APPLICATION

(11) **EP 4 461 699 A2**
(43) Date of publication of application: **13.11.2024**
(21) Application number: 24201504.8
(22) Date of filing: 30.03.2017
(51) Int. Cl.: B82Y 40/00

(54) **NANOSTRUCTURE ARRAY BASED SENSORS FOR ELECTROCHEMICAL SENSING, CAPACITIVE SENSING AND FIELD-EMISSION SENSING**

(30) Priority: 30.03.2016 US 201662315609 P
(62) Divisional of application: 17776629.2
(71) Applicant: Khalid, Waqas, Berkeley, CA 94704 (US)
(72) Inventor: Khalid, Waqas, Berkeley, CA 94704 (US)
(74) Representative: Kraus & Lederer PartGmbB

(57) **Abstract**

The present invention relates to utilizing individually addressable nanostructure arrays as nano electrodes for multianalyte electrochemical sensing via utilizing various electrochemical spectroscopy, capacitive and field emission techniques. In certain aspects, the invention provides devices and arrangements comprising at least two individually addressable nanostructures in an array on a substrate, and uses thereof. In other certain aspects, the invention features systems comprising the device and a chip holder, and further comprising hardware and software.

## Description

### RELATED APPLICATIONS

The present invention claims priority to U.S. Provisional Application No. 62/315,609, filed March 30, 2016, the entire contents of which is hereby incorporated by reference herein.

### FIELD OF THE INVENTION

The present invention relates to an electrical device comprising of individually addressable nanostructures in an array format for sensing of analytes using electrochemical spectroscopy, capacitance and field emission techniques. The said device can be used to manipulate, monitor and detect cells. The said device can also be used as a high resolution electrochemical camera.

### BACKGROUND OF THE INVENTION

Electrochemical spectroscopy is a powerful technique to monitor chemicals in liquids especially solutions. It is frequently used in biological sensing. Various techniques fall under electrochemical spectroscopy including voltammetry, amperometry, cyclic voltammetry, fast scan cyclic voltammetry, electrochemical impedance spectroscopy, stripping voltammetry etc. Various sizes, shapes and materials of electrodes are utilized in electrochemical measurements to improve the signal to noise ratio. Smaller electrode sizes with higher surface area are preferred as they have higher sensitivity. An array of individually addressable nanostructures is a perfect solution for electrodes to perform sensitive, fast and multianalyte electrochemical spectroscopy. Such an array was reported in WO2013001076, incorporated by reference in its entirety herein. Such nanostructures can be packed in a minute space and hence provide higher spatial and sensing resolution for various sensing applications. The nanostructures can be functionalized with different chemicals as well, for example as described in WO2013132352, incorporated by reference in its entirety herein. The devices can be used for a unique sensing scheme as well allowing the realization of electrochemical cameras for imaging materials based on their chemical compositions.

Capacitive sensing is also employed by researchers to detect gases and analytes in solids, liquids and gases. The size and functionalization of the capacitor electrodes determines the sensitivity and selectivity of the detection method of analytes respectively. When an analyte comes between the electrodes of a capacitor or a super capacitor, it cases change in the capacitance of the system which can then be measured. This is a powerful technique to detect the size of analytes as well. Hence, we can employ capacitance tomography using such devices.

Moreover, field emission based sensing of gases and impurities in gases can also be performed using such an array of nanostructures. When a voltage is applied between two electrodes composed of nanostructures, field emission (movement of electrons from one electrode to another via air or vacuums) occurs. When gas molecules or impurities or analytes come between the electrodes, ionization of gases and other matter occurs causing variation of the field emission current. This variation can be detected using appropriate electronics and software. The material of the electrodes, the distance between the electrodes and the voltage applied are all factors contributing to the sensitivity of this methods.

Presently, the electrodes are either large or the composite nanomaterial electrodes are formed in such a way that they are not bound on a surface. Hence, they tend to peel off during electrochemical measurements. Furthermore, forming densely packed electrodes for capacitive sensing is a challenge and the fabrication can be cumbersome. Similar issues occur when field emission devices are formed. If they are too far apart, performing field emission in air is challenging due to arcing in air. However, if the electrodes are close enough such that the mean free path of air molecules is comparable to the distance between the electrodes, then such field emission devices can be realized. Accordingly, there remains a need in the art for improved field emission devices.

### SUMMARY OF THE INVENTION

The present invention is based upon novel and inventive methods of utilizing individually addressable nanostructure arrays as nano electrodes for multianalyte electrochemical sensing via utilizing various electrochemical spectroscopy, capacitive and field emission techniques. The materials for nanostructures include carbon nanotubes which have excellent electrical, thermal and mechanical properties.

In a first aspect, the present invention provides an arrangement of at least two individually addressable nanostructures (207) in an array on a substrate (201), wherein the substrate (201) is non-conducting, wherein there are conducting electrical portions (208) within the substrate, wherein the conducting electrical portions form electrical contacts with the nanostructures (207) forming the individually addressable nanostructures in an array, wherein the nanostructures (207) are individually connected with conductive paths (403) on the first face (202) of the non-conducting substrate (201) and conductive structures (210) in a second substrate (209) via the conductive portion (208) in the first substrate (201) wherein the said nanostructures (207) are covered with a medium (3000), and wherein when a voltage (900) is applied between the at least two nanostructures (207), an electric or electromagnetic field is generated between the said nanostructures and a capacitance (700) is formed between the nanostructures.

In one embodiment, the electrical field results in movement of charged material (800) between the nanostructures. In one embodiment, each nanostructure (207) has a base size (2210), wherein the base size (2210) ranges from about 1-1 000,000 nm. In one embodiment, the height (2220) ranges from about 10 - 1 000 000 nm. In one embodiment, the nanostructures (207) comprise one or more nano-materials. In one embodiment, the nanostructures (207) are selected from the group consisting of: nanotubes, nanofibers, nano rods and nano wires. In one embodiment, the nanostructures (207) are selected from carbon nanotubes, carbon nanofiber, silicon nanowires, zinc oxide Nano rods. In one embodiment, the distance, (2213) is the gap between each nano-material that range from 1-100 nm. In one embodiment, the at least two nanostructures (207) are separated from each other by a distance (800), wherein the distance (800) ranges from 1-100000 nm. In one embodiment, the at least two nanostructures are charged with a positive charge or negative charge by the electrical portion in substrate. In one embodiment, each nanomaterial has a base size (2212) of 1-100 nm and height (2211) that ranges from 1-1 000,000 nm. In one embodiment, the medium (3000) is a solid surface or a liquid or a gas. In one embodiment, the medium is stationary or in a flow. In one embodiment, the medium is selected from the group consisting of vacuum, air, gas mixtures, polymer, ceramics, silicon, semiconductors, metals, silicone, quarts, mica, Teflon, oil, solutions and liquids mixtures. In one embodiment, the medium (3000) is at least about 1- 500000 nm thicker than the height of the nanostructures. In one embodiment, the voltage (900) can be applied between the nanostructures and an external electrode. In one embodiment, the material of the external electrode can be selected from the group consisting of metals, composite materials, semiconductors, conducting polymers, and silver/silver chloride. In one embodiment, the nanostructure array can be charged with constant charge or current. In one embodiment, the nanostructure array can be charged with alternating charge or current. In one embodiment, capacitance (700) is formed between the nanostructures and the direction of the electrical field is dependent on the polarity of the voltage (900) applied. In one embodiment, the medium further comprises an analyte (600) in the medium (3000). In another embodiment, the analyte (600) comprises an impurity in the medium. In one embodiment, the size of the analyte is from 1 angstrom to 1 mm; preferably from 1 nm to 1000 nm; most preferably from 1 angstrom to 10 nm. In one embodiment, the analyte is selected from the group consisting of ions, cells, nano particles DNA, RNA, bio-molecules, polymers, ceramics, metals, gases, bacteria, viruses, vapors, and toxins. In one embodiment, the analyte is a chemical. In one embodiment, the chemical is detected using electrochemical spectroscopy due to electrochemical changes or impedance changes caused by the analyte in the medium. In one embodiment, the analyte is a chemical that can be detected using capacitance changes due to dielectric constant changes in the medium caused by the analyte. In one embodiment, the analyte is a chemical that can be detected using field emission sensing as the analyte is ionized by the field emission causing a change in the properties of the medium.

In another aspect, the present invention features a device (300) comprising at least two individually addressable nanostructures (207) in an array on a substrate (201), wherein the substrate (201) is non-conducting with conducing electrical portions (208) within the substrate, wherein the conducting electrical portions form electrical contacts with the nanostructures (207) forming the individually addressable nanostructures in an array, wherein the nanostructures (207) are individually connected with conductive paths (403) on the first face (202) of the non-conducting substrate (201) and conductive structures (210) in a second substrate (209) via the conductive portion (208) in the first substrate (201) wherein the said nanostructures (207) are covered with a medium (3000), and wherein when a voltage (900) is applied between at least two nanostructures (207), an electric or electromagnetic fieldis generated between the said nanostructures and a capacitance (700) is formed between the nanostructures.

In one embodiment, the electrical field results in movement of charged material (800) between the nanostructures. In one embodiment, at least one nanostructures (207) in the array can be charged with a first charge and at least a second nanostructures (207) in the array can be charged with a second charge. In one embodiment, the electrical interaction between the first set and the second set of nanostructures will generate a first electrical signal, wherein external perturbation or presence of analyte (600) in the medium (3000) creates a change the electric field. In one embodiment, the electrical interaction between the first set and the second set of nanostructures will generate a first electrical signal, wherein external perturbation or presence of analyte (600) in the medium (3000) creates a change the capacitance (700). In one embodiment, the electrical interaction between the first set and the second set of nanostructures will generate a first electrical signal, wherein external perturbation or presence of analyte (600) in the medium (3000) creates a change the flow of charged materials between the said two nanostructures causing a change that can generate a second electrical signal. In another embodiment, the analyte (600) comprises an impurity in the medium. In one embodiment, such first and second signals from the nanostructures can be can be utilized as pixilated sensor signals for electrochemical sensing, using external circuit connected to the device (300). In one embodiment, such first and second signals from the nanostructures can be utilized as pixilated sensor signals for capacitive sensing, using external circuit connected to the device (300). In one embodiment, such first and second signals from the nanostructures can be utilized as pixilated sensor signals for field emission based sensing using external circuit connected to the device (300). In one embodiment, the device as described in any one of the aspects or embodiments herein is for use as an electrochemical, capacitive and/or field emission sensor array. In one embodiment, the nanostructures act as a nano electrode array for electrochemical detection of analytes (600) in the medium (3000), wherein the arrangement is employed is as capacitive sensing device wherein the nanostructures act as nano electrode array for capacitive sensing of analytes (600) in the medium (3000), and wherein the arrangement is employed as a field emission based sensing device wherein the nanostructures act as nano electrode array for field emission based sensing of analytes (600) in the medium (3000). In another embodiment, the analyte (600) comprises an impurity in the medium. In one embodiment, the nanostructures are functionalized. In one embodiment, the functionalization is performed via covalent functionalization, surface adsorption, electro-polymerization or electrochemical deposition. In one embodiment, the functionalization of the nanostructures enhances the charging on the nanostructures. In one embodiment, the functionalization of the nanostructures enhances the sensing of multianalyte simultaneously. In one embodiment, the functionalization is with chemicals and/or via covalent functionalization, surface adsorption, electrochemical deposition for enhanced sensing of multianalyte simultaneously In one embodiment, the said conductive portion in the insulating layer is photovoltaic (208). In one embodiment, the said conducting portion (208) produces electricity when the material is exposed to light. In one embodiment, exposure to light allows energy harvesting from electromagnetic waves to electricity for self-powering device.

In another embodiment, the present invention provides a system (4000) comprising of the device (300) as described in any of the aspects and embodiments herein, and a chip holder (4401). In one embodiment, the chipholder (4401) provides at least one electrical contact with the nanostructure array device (300). In one embodiment, the chipholder (4401) provides microfluidics for medium (3000) around the nanostructure arrays (207). In one embodiment, the chipholder (4401) provides an electrical connection for external hardware (4402). In one embodiment, the external hardware (4402) comprises data acquisition and signal generation hardware electronics. In one embodiment, the hardware (4402) is connected to a software (4403) using a wired or wireless connection, and wherein the software (4403) process the data generated from the device (300).

In another aspect, the present invention features a system (4000) comprising a nanostructure array sensing device (300), a chip holder (4401), hardware (4402) and software (4403).

In one embodiment, the system can detect multianalyte simultaneously with high efficiency of detection and utilizes differential sensing, high surface area nano electrode arrays, electronics and software algorithms to improve sensing.

In another aspect, the present invention features a method of monitoring, detecting or manipulating of cells using the system as described in any one of the aspects and embodiments herein. In one embodiment, manipulation of cell includes cell poration, wherein an electric charge is delivered to the cell membrane (1401) using the nanostructure (207), wherein the electric charge causes a shock to the cell, and wherein the cell membrane open up (1404) at site-specifically at the nanostructure (207) used to deliver the electric charge. In one embodiment, one or more chemicals and/or analytes in a cell and around a cell can be detected, wherein the detection of chemicals and analytes include the intra cell analyte measurements, measurements of potentials and analytes across cell membrane, analyte measurement in the micro environment of the cells using electrochemical, capacitive and field emission techniques. In one embodiment, functionalized nanostructures (5207) are used to deliver chemicals inside cell without damaging the cell using electroporation, wherein the functional group on the nanostructure can be delivered inside the cell. In one embodiment, cell monitoring includes a cell that is monitored for movement, chemical and analyte excretion and intake using electrochemical, capacitive or field emission sensing, wherein the cell is a single cell in isolation in a medium (3000), wherein the cell is a single cell in a population of cells in a medium, or wherein the cell is in interaction with multiple other cells. In one embodiment, the detection cell includes detection of chemicals and analytes, wherein the chemicals and analytes are in the micro environment of single cell in the medium (3000), wherein the chemical activity of the single cell membrane can be detected with special resolution using individually addressable nanostructures, and wherein the cells are in vivo or in vitro.

In another embodiment, the present invention provides a method of multianalyte detection using the system as described in any of the aspects and embodiments herein. In one embodiment, multiple analytes can be detected simultaneously. In one embodiment, multiple analytes can be detected in real time. In one embodiment, detection is carried out using one or more of electrochemical spectroscopy, capacitive sensing or field emission sensing. In one embodiment, the size of the analyte is determined. In one embodiment, the concentration of analytes is detected. In one embodiment, the system further comprises remote computing and data storage locations. In one embodiment, the system further performs data analyses. In one embodiment, the data analysis from multiple systems can be analyzed simultaneously.

In another embodiment, data acquisition comprises of data acquisition /connection port, amplifier/analog circuitry, ADC, microcontroller and communication portal Wherein hardware for signal generation comprises of input settings variables, microcontrollers, digital potentiometer, amplifiers/analog circuitry/buffers and output port/connector. In another embodiment, data processing software can process the data generated from the device as described herein, and comprises at least one of the following processes of raw data manipulation and allows graphical representation of raw data utilizes machine learning algorithm is capable of comparison of new data with learned data over time or data in a database and produce analysis output of the data.

In one embodiment, the device as described in any of the aspects and embodiments herein, can be used as an electrochemical camera for imaging of the chemical compositions of analytes in liquids, gases or surfaces, membranes via electrochemical spectroscopy, capacitive sensing or using field mission sensing methods. In one embodiment, the device and/or system as described in any of the aspects and embodiments herein can connect with a remote computing location (cloud) and data from multiple systems and devices can be analyzed simultaneous to allow comparison of data from multiple systems, creating a snapshot of the ecosystem.

The following aspects are preferred embodiments of the invention:
1. An arrangement of at least two individually addressable nanostructures (207) in an array on a substrate (201),
   wherein the substrate (201) is non-conducting, wherein there are conducting electrical portions (208) within the substrate,
   wherein the conducting electrical portions form electrical contacts with the nanostructures (207) forming the individually addressable nanostructures in an array,
   wherein the nanostructures (207) are individually connected with conductive paths (403) on the first face (202) of the non-conducting substrate (201) and conductive structures (210) in a second substrate (209) via the conductive portion (208) in the first substrate (201)
   wherein the said nanostructures (207) are covered with a medium (3000), and
   wherein when a voltage (900) is applied between the at least two nanostructures (207), an electric or electromagnetic field is generated between the said nanostructures and a capacitance (700) is formed between the nanostructures.
2. The arrangement of aspect 1, wherein the electrical field results in movement of charged material (800) between the nanostructures.
3. The arrangement according to aspect 1 wherein each nanostructure (207) has a base size (2210), wherein the base size (2210) ranges from about 1-1 000,000 nm.
4. The arrangement according to aspect 1, wherein the height (2220) ranges from about 10 - 1 000 000 nm.
5. The arrangement according to aspect 1, wherein the nanostructures (207) comprise one or more nano-materials.
6. The arrangement according to aspect 1, wherein the nanostructures (207) are selected from the group consisting of: nanotubes, nanofibers, nano rods and nano wires.
7. The arrangement according to aspect 1, wherein the nanostructures (207) are selected from carbon nanotubes, carbon nanofiber, silicon nanowires, zinc oxide Nano rods.
8. The arrangement according to aspect 1, wherein the distance, (2213) is the gap between each nano-material that range from 1-100 nm.
9. The arrangement according to aspect 1, wherein the at least two nanostructures (207) are separated from each other by a distance (800), wherein the distance (800) ranges from 1-100000 nm.
10. The arrangement according to aspect 1, wherein the at least two nanostructures are charged with a positive charge or negative charge by the electrical portion in substrate.
11. The arrangement according to aspect 1, wherein each nanomaterial has a base size (2212) of 1-100 nm and height (2211) that ranges from 1-1 000,000 nm.
12. The arrangement of aspect 1 wherein the medium (3000) is a solid surface or a liquid or a gas.
13. The arrangement of aspect 1, wherein the medium is stationary or in a flow.
14. The arrangement according to aspect 1, wherein the medium is selected from the group consisting of vacuum, air, gas mixtures, polymer, ceramics, silicon, semiconductors, metals, silicone, quarts, mica, Teflon, oil, solutions and liquids mixtures.
15. The arrangement according to aspect 1, wherein the medium (3000) is at least about 1- 500000 nm thicker than the height of the nanostructures.
16. The arrangement according to aspect 2 wherein the voltage (900) can be applied between the nanostructures and an external electrode.
17. The arrangement of aspect 16, wherein the material of the external electrode can be selected from the group consisting of metals, composite materials, semiconductors, conducting polymers, and silver/silver chloride.
18. The arrangement according to aspect 1 wherein the nanostructure array can be charged with constant charge or current.
19. The arrangement according to aspect 1, wherein the nanostructure array can be charged with alternating charge or current.
20. The arrangement according to aspect 1, wherein capacitance (700) is formed between the nanostructures and the direction of the electrical field is dependent on the polarity of the voltage (900) applied.
21. The arrangement according to aspect 1, wherein the medium further comprises an analyte (600) in the medium (3000).
22. The arrangement according to aspect 21, wherein the size of the analyte is from 1 angstrom to 1 mm; preferably from 1 nm to 1000 nm; most preferably from 1 angstrom to 10 nm.
23. The arrangement according to aspect 21, wherein the analyte is selected from the group consisting of ions, cells, nano particles DNA, RNA, bio-molecules, polymers, ceramics, metals, gases, bacteria, viruses, vapors, and toxins.
24. The arrangement according to aspect 21, wherein the analyte is a chemical.
25. The arrangement according to aspect 24, wherein the chemical is detected using electrochemical spectroscopy due to electrochemical changes or impedance changes caused by the analyte in the medium.
26. The arrangement according to aspect 24, wherein the analyte is a chemical that can be detected using capacitance changes due to dielectric constant changes in the medium caused by the analyte.
27. The arrangement according to aspect 24, wherein the analytes is a chemical that can be detected using field emission sensing as the analyte is ionized by the field emission causing a change in the properties of the medium.
28. A device (300) comprising at least two individually addressable nanostructures (207) in an array on a substrate (201),
   wherein the substrate (201) is non-conducting with conducing electrical portions (208) within the substrate,
   wherein the conducting electrical portions form electrical contacts with the nanostructures (207) forming the individually addressable nanostructures in an array,
   wherein the nanostructures (207) are individually connected with conductive paths (403) on the first face (202) of the non-conducting substrate (201) and conductive structures (210) in a second substrate (209) via the conductive portion (208) in the first substrate (201)
   wherein the said nanostructures (207) are covered with a medium (3000), and
   wherein when a voltage (900) is applied between at least two nanostructures (207), an electric or electromagnetic field is generated between the said nanostructures and a capacitance (700) is formed between the nanostructures.
29. The device according to aspect 28 wherein the electrical field results in movement of charged material (800) between the nanostructures.
30. The device according to aspect 28, wherein at least one nanostructures (207) in the array can be charged with a first charge and at least a second nanostructures (207) in the array can be charged with a second charge.
31. The device according to aspect 28, wherein the electrical interaction between the first set and the second set of nanostructures will generate a first electrical signal,
   wherein external perturbation or presence of analyte (600) in the medium (3000) creates a change the electric field.
32. The device according to aspect 28, wherein the electrical interaction between the first set and the second set of nanostructures will generate a first electrical signal,
   wherein external perturbation or presence of analyte (600) in the medium (3000) creates a change the capacitance (700).
33. The device according to aspect 28, wherein the electrical interaction between the first set and the second set of nanostructures will generate a first electrical signal,
   wherein external perturbation or presence of analyte (600) in the medium (3000) creates a change the flow of charged materials between the said two nanostructures causing a change that can generate a second electrical signal.
34. The device according to aspect 31-33, wherein such first and second signals from the nanostructures can be can be utilized as pixilated sensor signals for electrochemical sensing, using external circuit connected to the device (300).
35. The device according to aspect 31-33, wherein such first and second signals from the nanostructures can be utilized as pixilated sensor signals for capacitive sensing, using external circuit connected to the device (300).
36. The device according to aspect 31-33, wherein such first and second signals from the nanostructures can be utilized as pixilated sensor signals for field emission based sensing using external circuit connected to the device (300).
37. The device according to aspect 28, for use as an electrochemical, capacitive and/or field emission sensor array.
38. The device according to aspect 37, wherein the nanostructures act as a nano electrode array for electrochemical detection of analytes (600) in the medium (3000),
   wherein the arrangement is employed is as capacitive sensing device wherein the nanostructures act as nano electrode array for capacitive sensing of analytes (600) in the medium (3000), and
   wherein the arrangement is employed as a field emission based sensing device wherein the nanostructures act as nano electrode array for field emission based sensing of analytes (600) in the medium (3000).
39. The device according to aspect 28 wherein the nanostructures are functionalized.
40. The device according to aspect 39, wherein the functionalization is performed via covalent functionalization, surface adsorption, electro-polymerization or electrochemical deposition.
41. The device according to aspect 39, wherein the functionalization of the nanostructures enhances the charging on the nanostructures.
42. The device according to aspect 39, wherein the functionalization of the nanostructures enhances the sensing of multianalyte simultaneously.
43. The device according to aspect 28, wherein the said conductive portion in the insulating layer is photovoltaic (208).
44. The device according to aspect 43, wherein the said conducting portion (208) produces electricity when the material is exposed to light.
45. The device according to aspect 44, wherein exposure to light allows energy harvesting from electromagnetic waves to electricity for self-powering device.
46. A system (4000) comprising of the device (300) of aspect 28 and a chip holder (4401).
47. The system of aspect 46, wherein the chipholder (4401) provides at least one electrical contact with the nanostructure array device (300).
48. The system of aspect 46, wherein the chipholder (4401) provides microfluidics for medium (3000) around the nanostructure arrays (207).
49. The system of aspect 46, wherein the chipholder (4401) provides an electrical connection for external hardware (4402).
50. The system of aspect 49, wherein the external hardware (4402) comprises data acquisition and signal generation hardware electronics.
51. The system of aspect 49, wherein the hardware (4402) is connected to a software (4403) using a wired or wireless connection, and
   wherein the software (4403) process the data generated from the device (300).
52. A system (4000) comprising a nanostructure array sensing device (300), a chip holder (4401), hardware (4402) and software (4403).
53. A method of monitoring, detecting or manipulating of cells using the system of aspect 46.
54. The method of aspect 53 wherein manipulation of cell includes cell poration,
   wherein an electric charge is delivered to the cell membrane (1401) using the nanostructure (207),
   wherein the electric charge causes a shock to the cell, and
   wherein the cell membrane open up (1404) at site-specifically at the nanostructure (207) used to deliver the electric charge.
55. The method of aspect 53-54 wherein one or more chemicals and/or analytes in a cell and around a cell can be detected,
   wherein the detection of chemicals and analytes include the intra cell analyte measurements, measurements of potentials and analytes across cell membrane, analyte measurement in the micro environment of the cells using electrochemical, capacitive and field emission techniques.
56. The methods of aspect 53-54 wherein functionalized nanostructures (5207) are used to deliver chemicals inside cell without damaging the cell using electroporation,
   wherein the functional group on the nanostructure can be delivered inside the cell.
57. The method of aspect 53 wherein cell monitoring includes a cell that is monitored for movement, chemical and analyte excretion and intake using electrochemical, capacitive or field emission sensing,
   wherein the cell is a single cell in isolation in a medium (3000),
   wherein the cell is a single cell in a population of cells in a medium, or
   wherein the cell is in interaction with multiple other cells.
58. The method of aspect 53 wherein the detection cell includes detection of chemicals and analytes, wherein the chemicals and analytes are in the micro environment of single cell in the medium (3000),
   wherein the chemical activity of the single cell membrane can be detected with special resolution using individually addressable nanostructures, and wherein the cells are in vivo or in vitro.
59. A method of multianalyte detection using the system of aspect 46.
60. The method of aspect 59, wherein multiple analytes can be detected simultaneously.
61. The method of aspect 59, wherein multiple analytes can be detected in real time.
62. The method of aspect 59, wherein detection is carried out using one or more of electrochemical spectroscopy, capacitive sensing or field emission sensing.
63. The method of aspect 59, wherein the size of the analyte is determined.
64. The method of aspect 59, wherein the concentration of analytes is detected.
65. The system of aspect 46, wherein the system further comprises remote computing and data storage locations.
66. The system of aspect 65, wherein the system further performs data analyses.
67. The system of aspect 65, wherein the data analysis from multiple systems can be analyzed simultaneously.

### BRIEF DESCRIPTION OF THE DRAWINGS

Figure 1 illustrates a flow-chart schematically illustrating a method of manufacturing a device comprising of nanostructures according to embodiments in this application and as described in WO2013001076.
Figure 2 a-d illustrates enlarged cross-sectional views of the device, where each view corresponds to a stage of the manufacturing process according to the method of FIG. 1
Figure 3 illustrates a perspective view of an embodiment of the device according to the invention
Figure 4 a-c illustrates a cross-sectional side view of exemplary embodiments of the device according to the present invention
Figure 5 illustrates figure 4c
Figure 6 illustrates a flow-chart for a chip holder (4401), hardware (4402) and software (4403) that form a system (4000) as described in the embodiment in this document
Figure 7 illustrates details of two adjacent nanostructures with difference respective charges

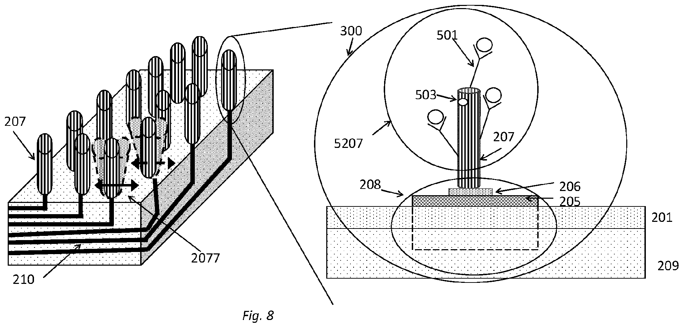

Figure 8 illustrates individually addressable nanostructure array and how each nanostructure us functionalized by a functional group.
Figure 9 illustrates a cross-section view and top view of nanostructure array based device where the nanostructures are covered in a medium (300) with impurities (800) and the individual nanostructures are charged with different respective charges
Figure 10 illustrates the system (4000) with a nanostructure array device (3000) in a chip holder (4401) that is connected to hardware (4402) and the hardware is connected to a software (4403) on a computer or mobile device
Figure 11 illustrates a real world realization of device (4000) as an example
Figure 12 illustrates the device (4000) as electrochemical camera
Figure 13 illustrates charged nanostructure array functionalized with various groups in a medium (300) with analyte (600) and charged material (800)
Figure 14 illustrates a cell on nanostructure array in a medium where the cell is being manipulated, monitored, analytes and cell is being detected
Figure 15 (a-o) shows scan electron (SEM) micrographs of nanostructures composed of carbon nanotubes (CNTs) in various shapes and sizes
Figure 16 illustrates an SEM micrograph of CNT nanostructures functionalized with zinc oxide nano-rods
Figure 17 illustrates array of nanostructures where opposite voltage is applied between adjacent nanostructures causing field emission (701) between the nanostructures
Figure 18 illustrates a graph where breath is detected using field emission between two nanostructures in an experiment
Figure 19 illustrates a graph where breath is detected using field emission between two nanostructures in another experiment.

### DETAILED DESCRIPTION

The present invention is based upon novel and inventive methods of utilizing individually addressable nanostructure arrays as nano electrodes for multianalyte electrochemical sensing via utilizing various electrochemical spectroscopy, capacitive and field emission techniques.

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

The indefinite articles "a" and "an," as used herein in the specification and in the claims, unless clearly indicated to the contrary, should be understood to mean "at least one."

A range includes each individual member. Thus, for example, a range of 100 nm refers to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 nm.

It should also be understood that, unless clearly indicated to the contrary, in any methods claimed herein that include more than one step or act, the order of the steps or acts of the method is not necessarily limited to the order in which the steps or acts of the method are recited.

Exemplary embodiments are described herein with reference to illustrations that are schematic illustrations of idealized embodiments. As such, variations from the shapes of the illustrations as a result, for example, of manufacturing techniques and/or tolerances, are to be expected.

The present invention features a novel and inventive device (300) comprising nanostructures (207) arranged in an array on first nonconductive substrate (201), with conductive portions (208) wherein the nanostructures (207) are individually connected with conductive paths (403) and conductive structures (210) in a second substrate (209) via the conductive portion (208) in the first substrate (201), wherein the device (300) is employed as electrochemical sensor array, wherein the nanostructures act as nano electrode array for electrochemical detection of analytes in a medium; wherein the device (300) is employed is as capacitive sensing device wherein the nanostructures act as nano electrode array for capacitive sensing of analytes in a medium; wherein the device (300) is employed as a field emission based sensing device wherein the nanostructures act as nano electrode array for field emission based sensing of analytes in a medium.

The present invention also features a system comprising the device (300), a chip holder (4401) device that provides at least electrical contacts with the nanostructure array device (300) and microfluidics for gas or liquids around the nanostructure arrays and connection for external electrical connections for hardware (4402) comprising: data acquisition and signal generation hardware, wherein data acquisition comprises of data acquisition, connection port, amplifier, analog circuitry, ADC, microcontroller and communication portal, wherein hardware for signal generation comprises of input settings variables, microcontrollers, digital potentiometer, amplifiers, analog circuitry, buffers and output port and connectors.

In one embodiment, the data processing software (4403) can process the data generated from the device (300) that comprises of at least one of the following processes of raw data manipulation and allows graphical representation of raw data utilizes machine learning algorithm is capable of comparison of new data with learned data over time or data in a database and produce analysis output of the data.

The system (4000) comprising of the nanostructure array sensing device, chip holder device, hardware and software for data processing as described herein is utilized, for example, for electrochemical, capacitance and field emission sensing applications.

The said devices can be used for capacitive sensing as they act as super capacitors when charged due to high surface area, small electrode size and small gap between the electrodes.

The said devices can also be used as field emission based sensing device due to close proximity of the nanostructures arranged on a substrate along with excellent field emission properties of nanostructures composed of material like carbon nanotubes, silicon carbide nanowires etc.

The said device can have nanostructure based arrays of individually addressable electrodes integrated with a chip holder that can provide electrical connection to the nanostructure arrays along with microfluidics to allow exposure and interaction of solids, liquids or gases with the nanostructure arrays. The chip-holder also incorporates hardware such as a multi-channel potentiostat that can generate signals and acquire data from the nanostructure array and transmit it to a software via hardwire or wirelessly. The device also comprises of software capable of receiving the data and graphing the data in real time or analyzing the data and providing a report. The software is capable of machine learning and artificial intelligence algorithms for providing an accurate analysis of the data from the nanostructure arrays.

The said device can be used as a multianalyte detection system for chemicals on solid surfaces, in liquid solutions or in gases. The device can be used to detect molecules, ions, DNA, RNA, proteins, nanoparticles, cells, sub cellular organelles, organic compounds, toxins and inorganic compounds. The devices can also be used to detect nanoparticles and differentiate the size of the nanoparticles. The nanostructures can be functionalized by different functional materials to allow multiple analyte detection with specificity. The devices can be used to monitor single cell in isolation, single cell in a population of cells, interaction of cells, micro environment of single cell and can provide special resolution of chemical activity of single cell membrane in vivo and in vitro.

The individual addressability of nanostructures allows variable signals to be sent to various nanostructures allowing multiple electrochemical detection techniques to be employed simultaneously for detection of analytes like chemicals, gases, and biomolecules etc. Similarly, applying different signals to the individually addressable nanostructures allow multi analyte detection using capacitive sensing methods and field emission sensing techniques. Combined with various functionalization on the nanostructures, numerous permutations and variations of the device use and applications are realized.

The said device can be used as electrochemical camera to image chemical compositions of surfaces and analytes with special resolution including size of the analyte, number and concentration of analytes, location of the analyte and material of the analyte.

The said device can also be utilized as a chemical camera using capacitive sensing method for chemical composition and size distribution of analytes including size of the analyte, number and concentration of analytes, location of the analyte and material of the analyte.

The said device can be used as a camera or artificial nose for sensing chemical composition of mixture of gases, volatile materials, explosives, signatures of materials and impurities etc. and their concentrations using field emission sensing techniques.

Accordingly, the present invention describes arrangements and devices that have surprising and unexpected uses. The arrangement and devices described in this document are innovative as they allow the use of nanomaterials like carbon nanotubes to perform sensing at high chemical and spatial resolution which is not possible in state of the art.

The utilization of the nanostructure device 300 in combination of the chip holder is unique because it provides electrical contacts to the device along with microfluidics, which has proven to be challenging at nanoscale. Moreover, the noise level for measurements at such small scale has proven to be difficult to overcome. Accordingly, unique ways to reduce the system noise are required, including but not limited to sealing the system to prevent evaporation, temperature monitoring, thermal stability, sensitive advanced electronics and advanced software to perform data analytics. Since the nanostructures have a huge surface area and are arranged close to each other, they can act as one device instead of an array, if same voltage is applied to adjacent electrodes. Moreover, the properties that make them super sensitive such as increased surface area and excellent electrical properties, can also make them super sensitive to pick up noise. Hence, special electronics is required to apply different potentials at different nanostructures and a powerful software is required to analyze data from multiple sensors simultaneously. There can be tens and hundreds and thousands of nanostructures in an array and to analyze data from all the devices simultaneously, a very powerful analytical software is required. Thus, to utilize the nanostructure array device for sensing application, a very special chip holder is required, as well as hardware and software to make useful measurements. The prior art has failed to provide the arrangements, devices and uses thereof, provided by the present invention. Indeed, the present invention is the result of years of experimentation to arrive at the appropriate setup, with optimal parameters to utilize the nanostructure device for multi analyte sensing. Further, the nothing in the prior art has predicted and solved the noise issues and physical changes (e.g. temperature and radiation) that can affect the signals. The present invention describes extensive experimentation and special precision to mitigate these issues, such that the system performs optimally.

In one aspect, the present invention describes an arrangement of at least two, at least, 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 60, at least 70, at least 80, at least 90, at least 100, at least 200, at least 300, at least 400, at least 500, at least 1000, at least 100,000, at least 1000,000, at least 1000,000,000, or more individually addressable nanostructures (207) in an array on a substrate (201). Preferably, the substrate (201) is non-conducting, and there are conducting electrical portions (208) within the substrate. Preferably, the conducting electrical portions form electrical contacts with the nanostructures (207) forming the individually addressable nanostructures in an array. Preferably, the nanostructures (207) are individually connected with conductive paths (403) on the first face (202) of the non-conducting substrate (201) and conductive structures (210) in a second substrate (209) via the conductive portion (208) in the first substrate (201). Preferably, the said nanostructures (207) are covered with a medium (3000), wherein when a voltage (900) is applied between the at least two nanostructures (207), an electric or electromagnetic field is generated between the said nanostructures and a capacitance (700) is formed between the nanostructures.

In one aspect, the present invention describes a device (300) comprising nanostructures (207) arranged in an array on first nonconductive substrate (201), with conductive portions (208) wherein the nanostructures (207) are individually connected with conductive paths (403) and conductive structures (210) in a second substrate (209) via the conductive portion (208) in the first substrate (201), wherein the device (300) is employed as electrochemical sensor array, wherein the nanostructures act as nano electrode array for electrochemical detection of analytes in liquids, wherein the device (300) is employed is as capacitive sensing device wherein the nanostructures act as nano electrode array for capacitive sensing of analytes in gases or liquids, wherein the device (300) is employed as a field emission based sensing device wherein the nanostructures act as nano electrode array for field emission based sensing of analytes in gases.

In another aspect, the present invention describes a chip holder device that provides at least electrical contacts with the nanostructure array device (300) and microfluidics for gas or liquids around the nanostructure arrays and connection for external electrical connections for hardware comprising of: data acquisition and signal generation hardware, wherein data acquisition comprises of Data acquisition /connection port, amplifier/analog circuitry, ADC, microcontroller and communication portal, wherein hardware for signal generation comprises of input settings variables, microcontrollers, digital potentiometer, amplifiers/analog circuitry/buffers and output port/connector.

Also encompassed by the present invention is data processing software that can process the data generated from the device (300) of above description, that comprises of at least one of the following processes of raw data manipulation and allows graphical representation of raw data utilizes machine learning algorithm is capable of comparison of new data with learned data over time or data in a database and produce analysis output of the data.

The system comprising of the nanostructure array sensing device, chip holder device and software for data processing as per above description is utilized for sensing application.

The system described above that can sense size by allowing for size detection in analytes from 1mm to 1 angstrom; preferably from 100 nm to 1 nm; most preferably from 1 nm to 1 angstrom; along with sensing chemicals by allowing chemical species detection of the analytes through processes of electrochemical spectroscopy, capacitive sensing or using field mission sensing methods.

The system described in above description that can sense concentration of analytes in a mixture by electrochemical spectroscopic detection, capacitive sensing or using field mission sensing methods.

The system in above description that can detect multianalyte simultaneously with high efficiency of detection and utilizes differential sensing, high surface area nano electrode arrays, electronics and software algorithms to improve sensing.

The system in above description that can perform cell poration; intra cell measurements, measurements across cell membrane, micro environment of the cells, deliver chemicals inside cell without damaging the cell.

The device in above description that contain nanostructures functionalized with chemicals via covalent functionalization, surface adsorption, electrochemical deposition for enhanced sensing of multianalyte simultaneously.

The device in above description that can be used as an electrochemical camera for imaging of the chemical compositions of analytes in liquids, gases or surfaces, membranes via electrochemical spectroscopy, capacitive sensing or using field mission sensing methods

The device and system described in the claims where multianalyte detection is performed simultaneously in real time using either one or a combination of electrochemical spectroscopy, capacitive sensing or using field mission sensing methods.

The devices can be used to monitor single cell in isolation, single cell in a population of cells, interaction of cells, micro environment of single cell and can provide special resolution of chemical activity of single cell membrane in vivo and in vitro.

The system as described in all the above claims can connect with cloud (remote computing and data storage) locations and perform data analyses.

The device and system described in the above claims can connect with a remote computing location (cloud) and data from multiple systems and devices can be analyzed simultaneous to allow comparison of data from multiple systems, creating a snapshot of the ecosystem.

The device in claim one where the said conductive portion in the insulating layer is photovoltaic (208); it produces electricity when the material is exposed to light, allowing energy harvesting from electromagnetic waves to electricity for self-powering device.

In an embodiment, in view of the above mentioned and other prior art, the present invention provides a method of utilizing individually addressable nanostructure arrays as nano electrodes for multianalyte electrochemical sensing via utilizing various electrochemical spectroscopy techniques. The materials for nanostructures include carbon nanotubes which have excellent electrical, thermal and mechanical properties.

In an embodiment, the said devices can be used for capacitive sensing as they act as super capacitors when charged due to high surface area, small electrode size and small gap between the electrodes.
n an embodiment, the said devices can also be used as field emission based sensing device due to good field emission properties of nanostructures composed of material like carbon nanotubes, silicon carbide nanowires etc.

In an embodiment, the said device can have nanostructure based arrays of individually addressable electrodes integrated with a chip holder that can provide electrical connection to the nanostructure arrays along with microfluidics to allow exposure and interaction of solids, liquids or gases with the nanostructure arrays.

In an embodiment, the chip-holder also incorporates hardware that can act as a multi-channel potentiostat that can generate signals and acquire data from the nanostructure array and transmit it to a software via hardwire or wirelessly.

In an embodiment, the device also comprises of software capable of receiving the data and graphing the data in real time or analyzing the data and providing a report.

In an embodiment, the software is capable of machine learning and artificial intelligence algorithms for providing an accurate analysis of the data from the nanostructure arrays.

In an embodiment where the nanostructure array is charged with constant charge and changing charges. Such an array of individually addressable nanostructures can be utilized as pixilated sensor for capacitive sensing.

In an embodiment where each nanostructure is 500 nm² base size and is composed nanomaterial chosen from but not limited to carbon nanotubes, nanofibers, nano rods. The distance between each nanomaterial element (in case of nanotubes, each nanotube in the nanostructure) is 5 nm. The two nanostructures are 500 nm apart from each other. The nanostructures are charged with a "+charge".

In an embodiment where the voltage is applied between the nanomaterials and an external electrode in the medium (3000). Suitable materials for top electrode that is not composed of nanostructures include transparent inorganic and organic conductive materials, chosen from the list but not limited to ITO (indium tin oxide), ATO (antimony tin oxide), tin oxide, PEDOT or other conductive polymers, and carbon nanotube or metal nanowire impregnated and composite materials.

In an embodiment where the nanostructures are composed of but not limited to carbon nanotubes or carbon nanofibers or Zinc oxide Nano rods or silicon nanowires or other nano materials.

In an embodiment, the nanostructures can be connected to external electrical circuit for power and signal processing software to obtain spatial resolution of analytes in the medium (3000).

In an embodiment where the nanostructures can be functionalized with various chemicals for enhancing the sensing capabilities.

In an embodiment, the medium material is chosen from the list but is not limited to this list including air, solutions, liquids, polymer, ceramics, oil, silicone, quarts, mica, Teflon, and strontium, buffer solutions and vacuum

In an embodiment, the medium is a dielectric such that it is an electrical insulator that can be polarized by an applied electric field. That means that dielectric materials are insulating materials or a very poor conductor of electric current. When the dielectric materials are placed in an electric field, no current flows through them they do not have loosely bound or free electrons that may drift through the material. Instead, electric polarization occurs.

In an embodiment, the nano structures can be formed on a substrate by evaporating a metal catalyst in areas patterned on the substrate using lithography, deep UV lithography or electron beam lithography techniques followed by chemical vapor deposition process to grow the nanomaterials at locations where the catalyst was deposited.

In an embodiment, the materials for the external electrode are chosen from the list but is not limited to this list including metals, conducting polymers like copper, platinum, titanium, conducting epoxy, silver paint, ITO (indium tin oxide), ATO (antimony tin oxide), tin oxide, PEDOT or other conductive polymers, and carbon nanotube or metal nanowire impregnated and composite materials, silver/silver chloride reference electrode.

In an embodiment, the substrate is chosen from the list but is not limited to this list including flexible substrate like polymers, silicones, polyamide,

In an embodiment, the substrate is chosen from the list but is not limited to this list including rigid substrates like silicon and silicon dioxide.

In an embodiment, the substrate is chosen from the list but is not limited to this list including a combination of solid and flexible of silicon islands in a polymer film connected with metal contact lines like small wires or thin films of metals

In an embodiment, the said device can be used as electrochemical camera to image chemical compositions of surfaces and analytes with special resolution.

In an embodiment, the said device can also be utilized as a chemical camera using capacitive sensing method for chemical composition and size distribution of analytes.

In an embodiment, the said device can be used as a camera or artificial nose for sensing chemical composition of mixture of gases and their concentrations using field emission sensing techniques.

In an embodiment, the system as described in this document that can connect with cloud (remote computing and data storage) locations and perform data analyses

In an embodiment, the device and system described in this document that can connect with a remote computing location (cloud) and data from multiple systems and devices can be analyzed simultaneous to allow comparison of data from multiple systems, creating a snapshot of the ecosystem

In an embodiment, the device in this document where the said conductive portion in the insulating layer is photovoltaic (208); it produces electricity when the material is exposed to light, allowing energy harvesting from electromagnetic waves to electricity for self-powering device

In an embodiment, hardware (4402) comprises of electronics not limited to, instrumentation electronics, operation amplifiers, transistors, diodes, resistors, capacitors, microcontrollers, inductors, data acquisition electronics, signal generation electronics

In an embodiment, the hardware (4402) also comprises of data acquisition electronics, wherein data acquisition comprises of data acquisition connection port, amplifiers, analog circuitry, analog to digital convertors (ADC), microcontroller and communication portal, wherein the hardware for signal generation comprises of input settings variables, microcontrollers, digital potentiometer, amplifiers, analog circuitry, buffers and output ports and connectors.

In another embodiment, software (4403) comprises of but not limited to, at least one of the following including an algorithm and/or a processes of raw data manipulation, graphical representation of raw data, utilizing machine learning algorithm and artificial intelligence algorithms, is capable of comparison of new data with learned data over time or data in a database and produce analysis output of the data generated from device (3000)

In an embodiment, the nanostructures (207) are composed of nanomaterials wherein the nanomaterials include but not limited to nanotubes, nanowires, nano-rods, carbon nanotubes, carbon nanofibers, graphene, silicon nanowires, zinc oxide Nano rods, composite materials etc.

In an embodiment, the individually addressable nanostructures in an array can range from at least 2 nanostructures. The range of nanostructures in the array can vary e.g. at least two, at least, 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 60, at least 70, at least 80, at least 90, at least 100, at least 200, at least 300, at least 400, at least 500, at least 1000, at least 100,000, at least 1000,000, at least 1000,000,000, or more.

In an embodiment, only 2 nanostructures are used for sensing application

In another embodiment, 10,000,000 nanostructures are used to scan a 300 mm long surface for detection

In another embodiment, 10,000 nanostructures are used to measure electrochemical or capacitance impedance spectroscopy to monitor a population of cells.

In an embodiment, nanomaterials are materials that are formed by sub-micron thick arrangement of elements and compounds including examples but not limited to, nanotubes, nano-rods, nanowires, 2D materials more specifically, but not limited to, graphene, carbon nanotubes, silicon nanowires etc.

In an embodiment, the nanostructures in an array have a base area, height and a distance between the nanostructures.

In another embodiment, the nanostructures are composed of nanomaterial that have their own respective base area, height and distance between nanomaterial within a nanostructure.

In an embodiment, the nanostructures are covered by a medium (3000) wherein the medium (3000) is at least about 1 nm - 500,000 um thicker than the height of the nanostructures.

In an embodiment, a method of monitoring or detecting or manipulation of cells using the system of described in this document.

In an embodiment, the device described above is used to manipulate a cell, wherein manipulation of cell includes cell poration, wherein an electric charge is delivered to the cell membrane (1401) using the nanostructure (207, wherein the electric charge causes a shock to the cell, wherein the cell membrane open up (1404) at site-specifically at the nanostructure (207) used to deliver the electric charge

In an embodiment, a method of wherein the chemicals and analytes in a cell and around a cell can be detected, wherein the detection of chemicals and analytes include the intra cell analyte measurements, measurements of potentials and analytes across cell membrane, analyte measurement in the micro environment of the cells using electrochemical, capacitive and field emission techniques.

In an embodiment, a device as described above, wherein functionalized nanostructures (5207) are used to deliver chemicals inside cell without damaging the cell using electroporation
wherein the functional group on the nanostructure can be delivered inside the cell.

In an embodiment, a device as described above, wherein cell monitoring includes a cell that is monitored for movement, chemical and analyte excretion and intake using electrochemical, capacitive or field emission sensing, wherein the cell is a single cell in isolation in a medium (3000), wherein the cell is a single cell in a population of cells in a medium, wherein the cell is in interaction with multiple other cells,

In an embodiment, a device as described above, wherein the detection cell includes detection of chemicals and analytes, wherein the chemicals and analytes are in the micro environment of single cell in the medium (3000)
wherein the chemical activity of single cell membrane can be detected with special resolution using individually addressable nanostructures, wherein the cells are in vivo, wherein the cells are in vitro.

In an embodiment, the said device can be used as a multianalyte detection system for chemicals on solid surfaces, in liquid solutions or in gases.

In an embodiment, the device can be used to detect molecules, ions, DNA, RNA, proteins, organic compounds and inorganic compounds.

In an embodiment, the devices can also be used to detect nanoparticles and differentiate the size of the nanoparticles, spatial location of the nanoparticles, the material of nanoparticles and concentration/ number of nanoparticles.
In an embodiment, the nanostructures can be functionalized by different functional materials to allow multiple analyte detection with specificity.

In an embodiment, the devices can be used to monitor single cell in isolation, single cell in a population of cells, interaction of cells, micro environment of single cell and can provide special resolution of chemical activity of single cell membrane in vivo and in vitro. In an embodiment, the individual addressability of nanostructures allows variable signals sent to various nanostructures allowing multiple electrochemical, capacitive and field emission detection techniques to be employed simultaneously for detection of analytes.

Similarly, in an embodiment, applying different signals to the individually addressable nanostructures allow multi analyte detection using electrochemical, capacitive and field emission sensing methods.

Similarly, in an embodiment, applying different signals to the individually addressable nanostructures allow multi analyte detection using electrochemical, capacitive and field emission sensing techniques.

In an embodiment, combined with various functionalization on the nanostructures, numerous permutations and variations of the device use and applications can be realized In an embodiment, a device as described above, multianalyte detection is achieved using the system

In an embodiment, a device as described above, wherein multiple analytes can be detected simultaneously. By multiple analytes is meant 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18, or more, 19 or more, 20 or more, 30 or more, 40 or more, 50 or more, 60 or more, 70 or more, 80 or more, 90 or more, 100 or more, 200 or more, 300 or more, 400 or more, 500 or more, up to a limitess mount of analytes.

In an embodiment, a device as described above, wherein multiple analytes can be detected in real time.

In an embodiment, a device as described above, wherein detection is carried out using one or more of electrochemical spectroscopy, capacitive sensing or field emission sensing.

In an embodiment, a device as described above, wherein the size of the analyte is determined.

In an embodiment, a device as described above, wherein the concentration of analytes is detected.

In an embodiment, a device as described above, wherein the system further comprises remote computing and data storage locations.

In an embodiment, chemicals are delivered into the cell using electroporation.

In an embodiment, the chemicals are delivered into the cell when the functional groups on the nanostructures come in contact with the cells.

In an embodiment, the chemicals are delivered to the cell when a small charge is applied to the functionalized nanostructure and the functional group is released from the nanostructure and absorbed by the cell.

In an embodiment, the cell is manipulated by electrical charge delivered to the cell by the nanostructures.

In an embodiment, the cell is manipulated by the functional group used to functionalize the nanostructures.

In an embodiment, the cell is manipulated by stimulants and chemicals delivered to the cell using the microfluidics in the chip holder.

In an embodiment, the cell is manipulated when it is forced to react to external stimuli like charge, chemicals, heat or light.

In an embodiment, the movement of the cell is monitored in a medium.

In an embodiment, the cell division is monitored in a medium.

In an embodiment, the transformation of a stem cell is monitored in a medium.

In an embodiment, the chemicals secreted by the cell is monitored in a medium.

In an embodiment, the chemicals absorbed/uptake by the cell is monitored in a medium.

In an embodiment, the movement of the cell is detected in a medium.

In an embodiment, the cell division is detected in a medium.

In an embodiment, the transformation of a stem cell is detected in a medium.

In an embodiment, the chemicals secreted by the cell is detected in a medium.

In an embodiment, the chemicals absorbed/uptake by the cell is detected in a medium.

In an embodiment, the system (4000) can sense the multianalyte simultaneously with high efficiency of detection is performed.

In an embodiment, the system (4000) can utilize differential sensing.

In an embodiment, the system (4000) can utilize the high surface area nanostructures as electrode arrays

In an embodiment, the system (4000) can utilize the hardware and sensitive electronics for high precision measurements and high signal to noise ratio measurements.

In an embodiment, the system (4000) can utilize the software algorithms to improve sensing.

### Advantages

Having described the various aspects and embodiments of the present invention, the following are exemplary advantages that are achieved by the present invention:
1- The device and system can perform multi analyte detection simultaneously in real time
2- The device and system can act as a chemical camera
3- The device and system can act as an artificial nose for chemical (gas) detection
4- The device and system can monitor single cell in isolation, single cell in a population of cells, interaction of cells, micro environment of single cell and can provide special resolution of chemical activity of single cell in vivo and in vitro
5- The device and system are mobile, hand help, robust and consume low energy
6- the device and system have passive sensors and hence consume very little power
8- The amount of sample required to perform testing is directly proportional to the size of sensors and since the devices are minute, (sub-micron to a few 10s of nano meter), multi analyte detection can be performed from very little samples, that can be costly or not very much of the sample is available at times
9- The device and system can perform chemical detection and size detection of analytes like nano particles along with concentrations/number of analytes, their material and spatial location.
10- The system can be connected to cloud networks for computational analyses, data storage.
11- The system can perform computations on data from multiple systems and multiple devices connected to systems that can be utilized for demographic studies of populations.
12- High Electric Field due to Nanostructures (carbon nanotube based) and the top electrode
13- Low power consumption by the device for operation as it's a passive device
14- This allows flexibility in design
15- Since the data is collected from higher number of sensors in the same location (other technologies have 1 sensor while we can pack multiple sensor in that same area), the resolution is increased and false signals can be prevented by smart algorithms that can eliminate outlier data-points
16- Ultra-super sensitive capacitance based fingerprint sensor can be realized
17- Spatial resolution of pressure at sub-micron level can be detected and mapped
18- Standalone, miniature, device thickness ranging from 100-1000 micro meters can utilized
19- Device can be integrated on flexible substrates.

It will be readily apparent to those skilled in the art that other suitable modifications and adaptations of the arrangements, devices and methods of the invention described herein may be made using suitable equivalents without departing from the scope of the invention or the embodiments disclosed herein. Having now described the present invention in detail, the same will be more clearly understood by reference to the following examples, which are included for purposes of illustration only and are not intended to be limiting of the invention.

### EXAMPLES

The following are the examples of some of the devices, systems and applications of the present invention.

In an example, the nanostructures can be made of carbon nanotubes, silicon nanowires, zinc oxide nano rods, silicon carbide nanowires, carbon nano fibers. In an example, the nanostructures can be 1 micrometer to 1 nanometer in size. In an example, the chip holder can be composed of plastics or metals resistant to corrosive chemicals. In an example, the chip holder can have pogo pins to connect with the chip containing nanostructure devices.

In an example, the hardware can have multichannel potentiostat as signal generator and data acquisition system. In an example, the software can run on stand-alone electronics, computers, laptops, mobile electronics or cloud computation systems. In an example, the software can have machine learning algorithms and/or artificial intelligence algorithms to perform data analysis.

In an example, a cell can be placed in a medium on top of the nanostructures. The cell can be any type of cell, and in certain examples can be a cell from a human subject. In certain examples, the cell is a stem cell. The stem cell can be placed in a medium on top of the nanostructures. For example, a stem cell can be placed in a medium on top of the nanostructures and electrochemical impedance spectroscopy can be used to monitor the transformation of stem cell to a bone cell.

In an example, a healthy bone cell can be placed in a medium on top of the nanostructures. In an example, a healthy bone cell can be placed in a medium on top of the nanostructures and exposed to radiation while the nanostructures can be used as capacitive tomography sensors to monitor the transformation of the bone cell from healthy cell to cancerous cell.

In an example, a voltage is applied between adjacent nanostructures that produce a field emission current in air. In an example, a voltage is applied between adjacent nanostructures that produce a field emission current in air and the nanostructures are exposed to human breath. The presence of different concentrations of gases in human breath along with water vapors change the field emission current, that can be detected by the hardware and software connected to the nanostructure device.

In an example, the voltage applied between at least two adjacent nanostructures is a DC voltage. In an example, the voltage applied between at least two adjacent nanostructures is an AC voltage. In an example, the voltage applied between at least two adjacent nanostructures ranges from about 10 V to -10 V. In an example, the voltage applied between at least two adjacent nanostructures ranges from about 5 V to -5 V. In an example, the voltage applied between at least two adjacent nanostructures ranges from about 1 V to -1 V. In an example, the voltage applied between at least two adjacent nanostructures ranges from about 1 mV to -1 mV. In an example, the frequency of the voltage applied between at least two adjacent nanostructures ranges from about 0.01 Hz to -100 MHz.

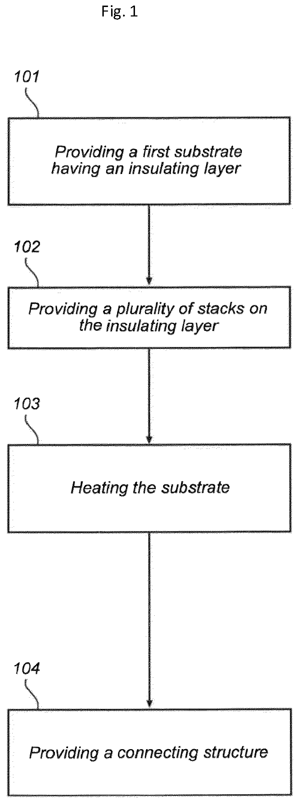

Figure 1 illustrates the method to manufacture nanostructure array as depicted in WO2013001076, incorporated by reference in its entirety herein.

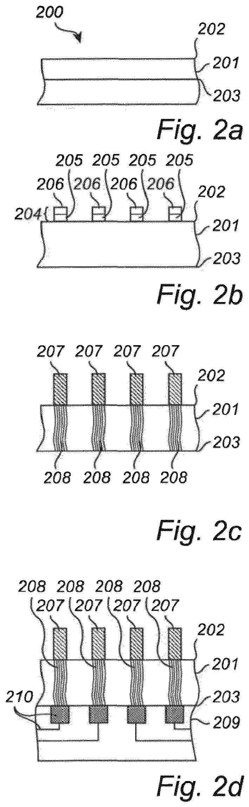

Figure 2 illustrates the steps of nanostructure array fabrication as described in WO2013001076, where in figure 2a. 201 is a non-conducting substrate with faces 202 and 203; Where in figure 2b, 204 is the metal stack where 205 is a conducting metal and 206 is a catalyst placed on ae 202 of substrate 201, where in figure 2c, 207 is the nanostructures and 208 is the conductive portion formed in the substrate 201, where in figure 2d, 209 is a second non-conducting substrate with electrical portion 210 providing an electrical contact to the conducting portions 208 at the face 203 of substrate 201.

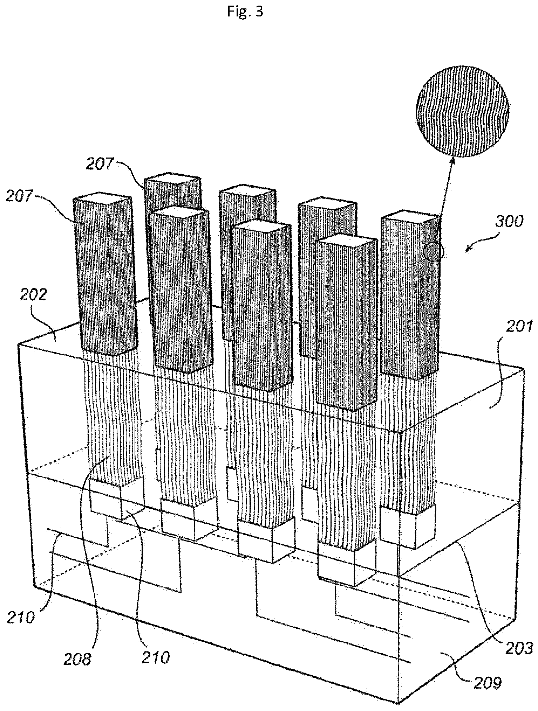

Figure 3 illustrates a device 300 comprising of nanostructures 207 on the face 202 of a non-conducting substrate 201 with conductive portions 208 and a second non-conducting substrate (209) with electrical portion 210 on face 203 of the first non-conducting substrate 201, as described in WO2013001076.

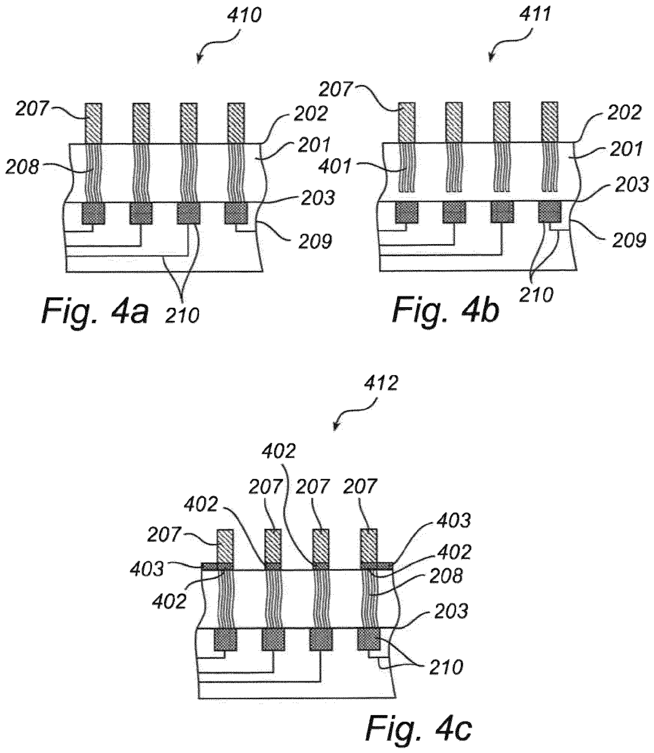

Figure 4 illustrates embodiments of devices 410, 411 and 412 as described in WO2013001076, wherein figure 4a, nanostructures 207 are connected with the conducting structures 210 via conducting portion 208 in non-conducting substrate 201, wherein figure 4b, nanostructures 207 are connected with the conducting structures 210 via conducting portion 208 in non-conducting substrate 201 via capacitive coupling, wherein figure 4c, nanostructures 207 are connected with the conducting structures 210 via conducting portion 208 in non-conducting substrate 201, and another electrical portion 403 provides a second contact pathway for nanostructures 207.

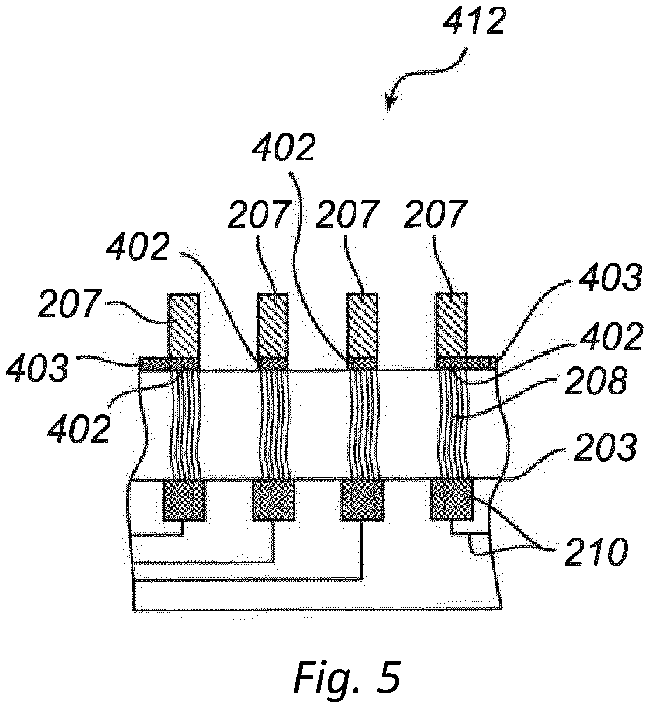

Figure 5 illustrates device 412 in more detail where nanostructures 207 are connected with the conducting structures 210 via conducting portion 208 in non-conducting substrate 201, and another electrical portion 403 provides a second contact pathway for nanostructures 207.

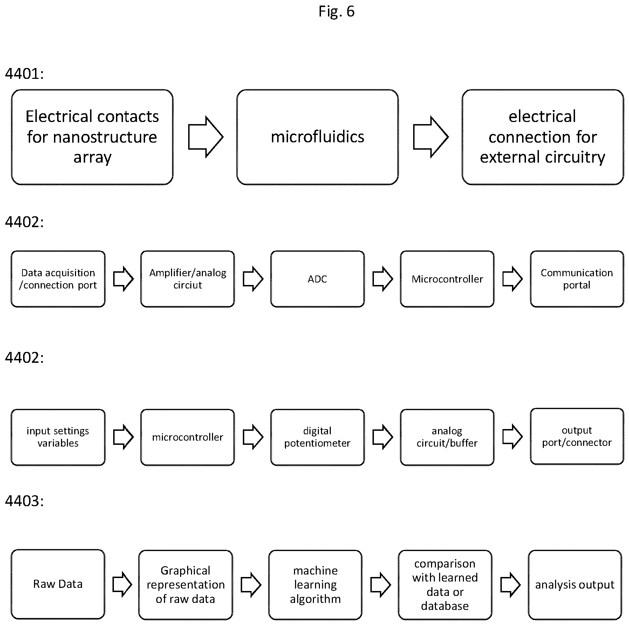

Figure 6 illustrates a flow-chart that lists of the requirements of a system (4000). The requirements are not limited to this list. The requirements include a chipholder (4401), hardware (4402) and software (4403) for utilizing nanostructures based device (300) as electrochemical, capacitive and field emission based sensors. The system (400) requires a chip-holder (4401) that can at least provide electrical connection (4411) to the nanostructure array along with microfluidics (4421) for interaction with solids, liquids or gases and connection for external electrical connections (4431) for hardware (4402) comprising of data acquisition and signal generation hardware, wherein data acquisition comprises of but not limited to data acquisition and connection port, amplifier and analog circuitry, ADC, microcontroller and communication portal. Also, the hardware (4402) comprises of but not limited to, signal generators wherein hardware for signal generation comprises of but not limited to, input settings variables, microcontrollers, digital potentiometer, amplifiers, analog circuitry, buffers and output port, wireless communication hardware and connectors. A data processing software (4403) that comprises of, but not limited to, raw data, graphical representation of raw data, machine learning algorithm, comparison with learned data or database, analysis output. The software can run on a computer or mobile electronic device or stand-alone electronics.

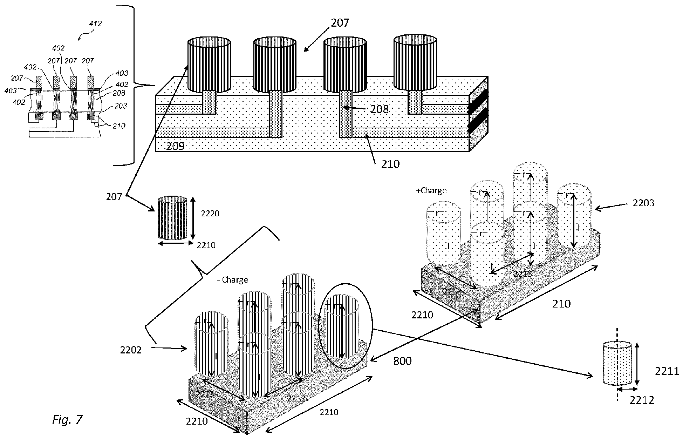

Figure 7 illustrates device (300) in various illustrations. Device (412) is represented in drawing where the nanostructures (207) is shown. Also shown are the nanostructures (207), that are connected to a conducting portion (208) in a non-conducting substrate (209) with conducting portions (210) that connect to the conduction portions (208). Also shown is details that nanostructures (207) have height (2220) and base size (2210). Also shown is that the nanostructures (207) are composed of nanomaterial (2203) that have the height (2211) and base size (2212). The gap between the nanostructures (207) is (800) while the gap between the nano materials is (2213). It is also shown that the nanostructures can be individually charged with different electrical charges wherein the electric charges being positive or negative.

Figure: 8 illustrates array of individually addressable nanostructures (207) and the device (300) wherein each nanostructure acts as a sensor or an electrode wherein the nanostructures (207) are functionalized with functional group (501) attached to it. The functionalized nanostructure (5207). Each nanostructure is connected to a non-conducting substrate (201) by a conducting portion (208) in the substrate. A nanoparticle (503) in the tip of the nanostructure is also shown that is composed of the material of the catalyst (206).

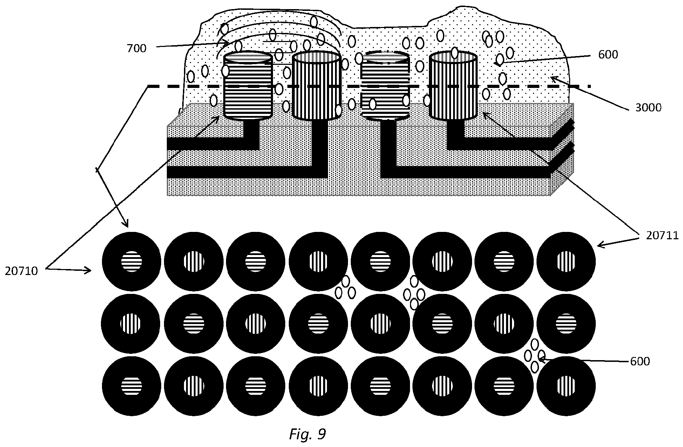

Figure 9 illustrates a cross section and top view of nanostructure array (207) in a medium (3000) containing analytes (600) where the nanostructures are charged for sensing application and a capacitance (700) is formed between the nanostructures.

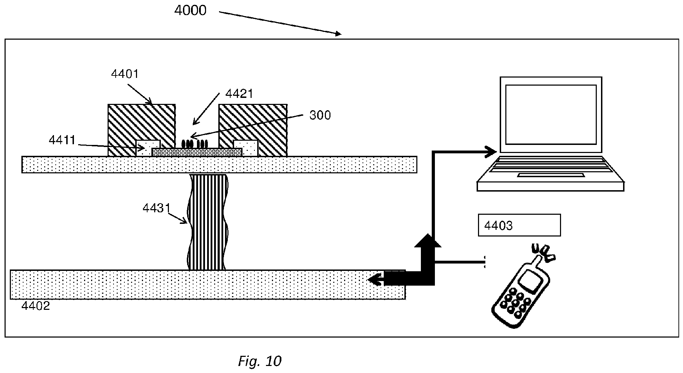

Figure 10 illustrates an example of the system (4000) where nanostructures array based device (300) is in a chip holder (4401) with electrical contacts (4411) and micro fluidics (4421). The chipholder is connected to hardware (4402) via electrical connections (4431). The hardware (4402 provides signals and perform data acquisition from the nanostructure array based device (300). The hardware (4402) is connected to a computer or a mobile device where the software (4403) performs analytics on the data and provides a report.

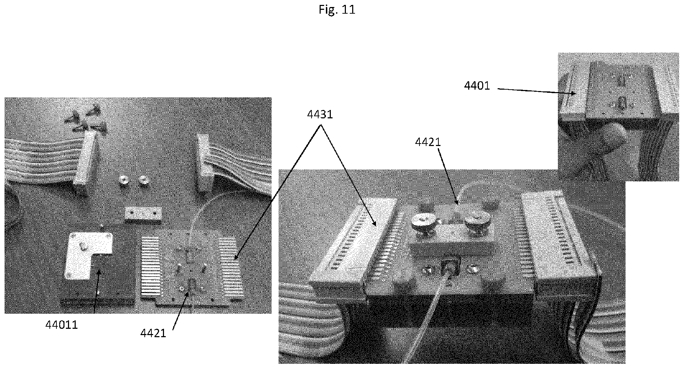

Figure 11 illustrates an example and a real-world manifestation of the system (4000) where nanostructures array based device (300) is in a chip holder (4401) with electrical contacts (4411) and micro fluidics (4421). The chipholder is connected to hardware (4402) via electrical connections (4431). The hardware (4402 provides signals and perform data acquisition from the nanostructure array based device (300). The hardware (4402) is connected to a computer or a mobile device where the software (4403) performs analytics on the data and provides a report.

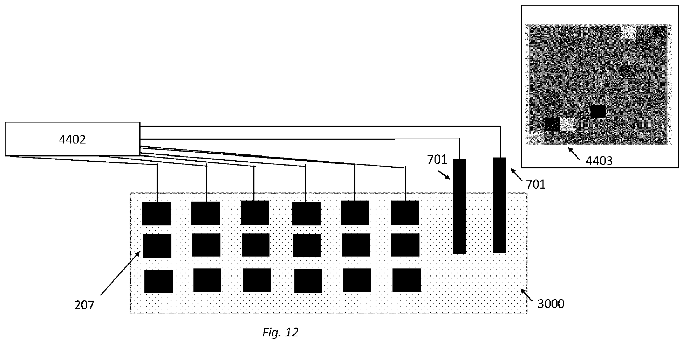

Figure 12 illustrates nanostructure (207) array used as sensors like an electrochemical sensor or a capacitive sensor or a field emission sensor array. Hardware (4402) can be used to generate and receive signals necessary for the sensor array. Hardware (4402) can be but not limited to a potentiostat for electrochemical spectroscopy. The resultant signals from each nanostructure can be utilized by software (4403) to prepare a chemical, capacitive or field emission based image of the analyte in the medium (3000). Each individually addressable nanostructure (207) acts as a single pixel for the imaging. The nanostructures act as working electrodes in the electrochemical spectroscopy process, capacitive sensing or field emission based sensing. External electrodes (701) can be used in examples like electrochemical sensing where (701) can be a counter or a reference or both electrodes in a three-electrode measurement setup. All the individually addressable nanostructures (207) work with shared external electrodes (701) like counter and reference electrodes.

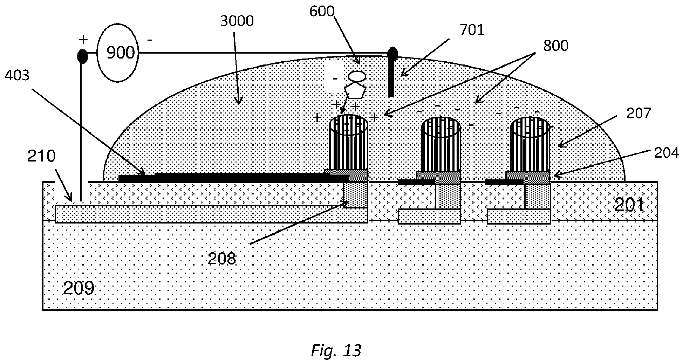

Figure 13 illustrates the arrangement of nanostructures (207) on a non-conducting substrate (201) with conducting portions (208). The conducting portions (208) are connection to conducting portions (210) in the non-conducting substrate (209). The nanostructures (207) are covered in a medium (3000) with analytes (600). When a voltage (900) is applied between the nanostructures and an external electrode (701) in the medium (3000), charge material (800) flows towards appropriate opposite charged nanostructures or external electrode. Such a method can be used to perform electrochemical detection of materials in a solution. Moreover, methods to functionalize the nanostructures after they are grown on a substrate are discussed by Waqas et al in patent [2]. The arrangement in this illustration can be used to further functionalize the nanostructures using electropolymerization where an electric potential is applied to the nano structures that need to be functionalized while an opposite polarity charge can be applied to the rest of the individually addressable nanostructures that are used as electrodes in this arrangement, to prevent non-specific binding and ensure functionalization on only the nano electrodes of interest. The arrangement in this illustration can be used to further functionalize the nanostructures using surface Adsorption (soak nanostructures with functionalization enzyme and other chemicals in solution. Proteins, antibodies and enzymes can be used to functionalized the nano structures using this method. Different nanostructures can be functionalized with different methods. Hence, all the techniques can be utilized to perform electrochemical spectroscopic detection of multiple analytes simultaneously.

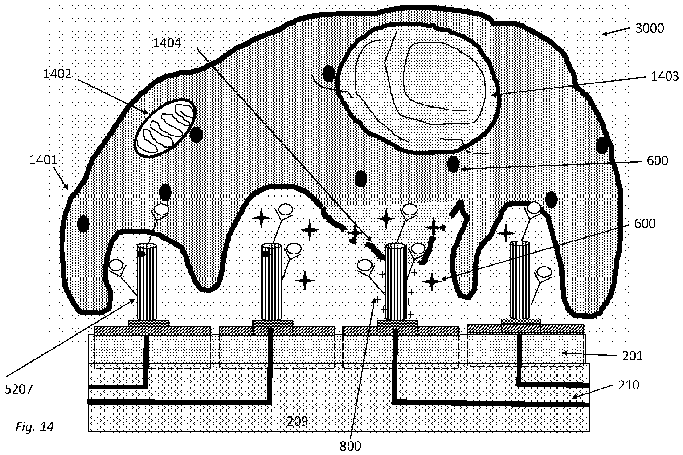

Figure 14 illustrates the details of the working principal of electrochemical sensor device discussed in figure 12 for cell applications. The cross-section of a single cell resting on functionalized nanostructure (5207) array in a medium (3000) is illustrated. The nanostructures (207) are arranged on a non-conducting substrate (201). To manipulate a cell, a positive charge is applied at one of the nanostructures, a site-specific electroporation is achieved (1404) where the cell membrane (1401) open up. This results in the flow of the fluids in the cell to flow out of the cell and mix with the medium (3000) at the location of the nanostructure where the charge is applied. This allows the analytes (600) inside the cell to come in proximity of the nanostructure and hence can be detected using electrochemical spectroscopy. Moreover, using the nanostructures adjacent to the electroporation site, chemical signals can be gathered, as depicted by stars (600) in the illustration. Interaction of functional groups on the nanostructures and intra cellular molecules can also be achieved using this method. By controlling the applied voltage on each of the nanostructures, an image the cell surface can be generated electrochemically. This technique can be used to monitor various cell behaviors including how cells move and divide. Hence, this figure illustrates methods where we can manipulation a cell, monitor its behavior and detect various chemicals inside and outside the cell in its micro environment, which is the environment close to the cell membrane in the medium. Some applications of this method can be used to detect cancer and monitor effects of the chemical compositions on a cell surface at a very early stage of cancer. It can also be used to monitor neuron degeneration when affected by Alzheimer's and Parkinson's disease. Moreover, we can monitor and detect specific chemical changes occurring in a stem cell micro environment before the cell transforms into a blood cell or a bone cell as an example.

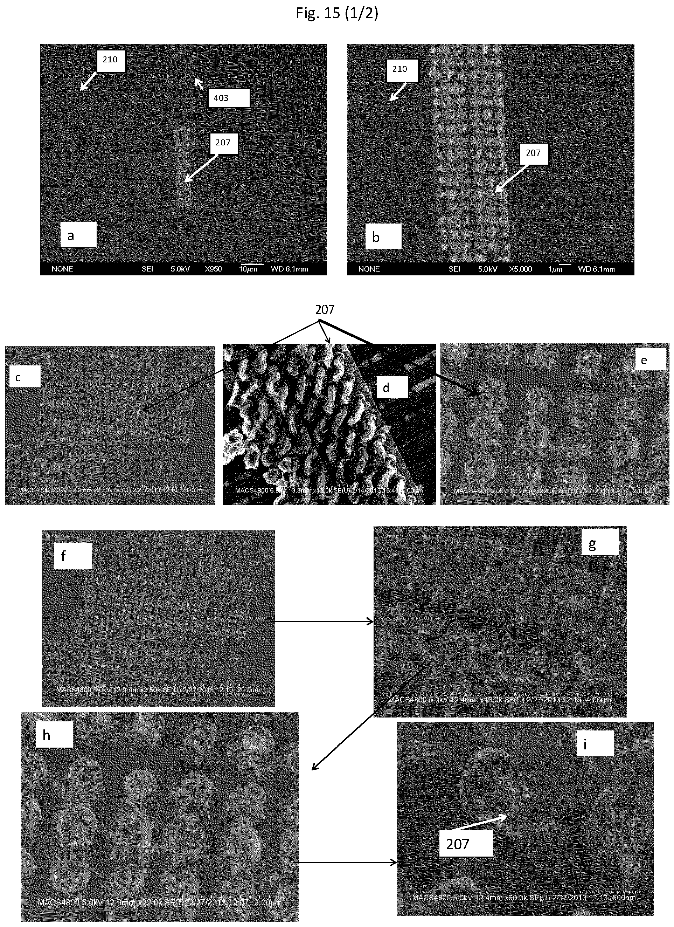

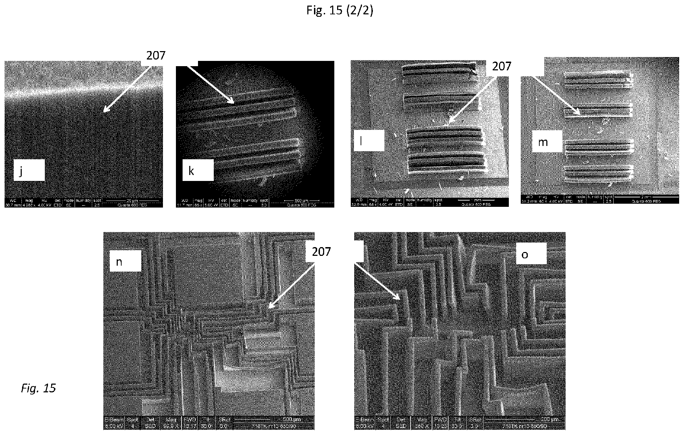

Figure 15: a-o Illustrates Scan Electron Microscopy (SEM) micrographs of various nanostructure arrays sizes. The length of the nanostructures can be controlled along with the base size of the nanostructures. A-b illustrate nanostructure (207) array along with conducting portion (210) buried in the silicon dioxide films. The nanostructures are 500 nm sq. in base size and are composed of nanomaterial, carbon nanotubes in these instances. The carbon nanotubes range from 500 nm to several microns tall. A top metal contact (403) can also be seen in figure 15a. Figure 15 a-i illustrates the various real world shapes and sizes of nanostructure (207) in arrays and the micrographs illustrate a 32 x 6 array of nano structures (figure 15 c and f) while others show zoomed in micrographs of carious nanostructures in the arrays. Figure 15 j-m illustrate longer baseline of the nanostructures composed of carbon nanotubes where the nanotubes are several hundreds of microns tall. Figure 15 n-o illustrate a complex arrangement of nanostructures where the nanostructures vary in base sizes and lengths across the arrangement. Thus, figure 15 illustrates various manifestations of the nanostructure based array devices.

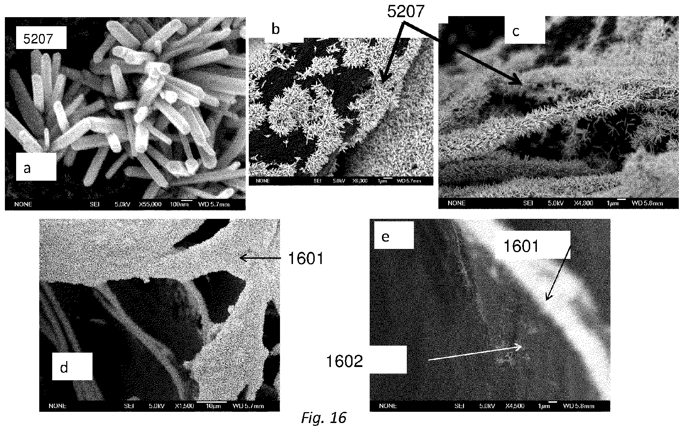

Figure 16: (a-e) illustrates a micrograph of carbon nanotube based nanostructures functionalized with zinc oxide nano rods (5207). Fig. 16-d illustrate the top view (1601) of the nanostructures composed of carbon nanotubes functionalized with zinc oxide nano-rods in abundance. Fig. 16-e illustrate the sidewalls (1602) of the nanostructures composed of carbon nanotubes where the functionalization is sparse.

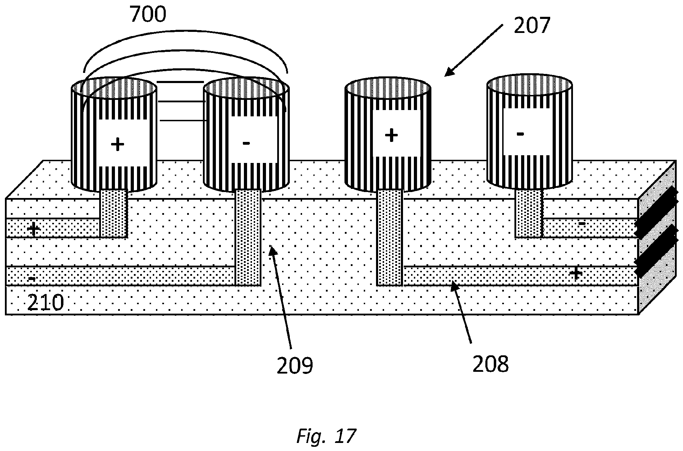

Figure 17 illustrates a cross section view of nanostructure array (207) where the nanostructures are charged and a capacitance (700) is formed between the nanostructures for sensing application. Field emission between the adjacent nanostructures occurs where electrons move from one nanostructures to the other as a result of the voltage applied. When gases pass by the nanostructures, ionization of the gasses occur, causing a variation in the field emission current. This variation can then be detected using the hardware (4402) and software (4403) described in this document. This is an example of utilizing the nanostructure array based device for field emission between the nanostructures (207) in order to sense the presence of gases as analytes (600) in a medium (3000) of air.

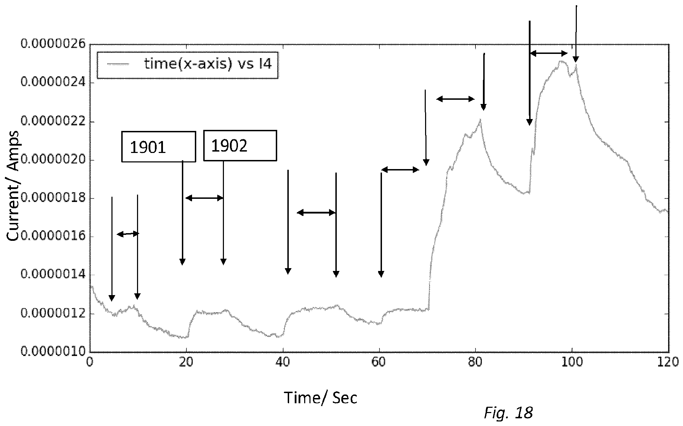

Figure 18 illustrates a graph showing the detection of breath using nanostructure array composed of carbon nanotubes (CNTS) as field emission based sensing devise. A voltage is applied between two adjacent nanostructures and the current (field emission current) is measured. The arrows (1901) indicate the time when the CNT nanostructures are exposed to human breath and the arrow (1902) indicates the time when the exposure to human breath was removed. It can be see that the field emission current (y axis) between the nanostructures increases when the nanostructures are exposed to human breath. Hence, gases can be detected using this technique.

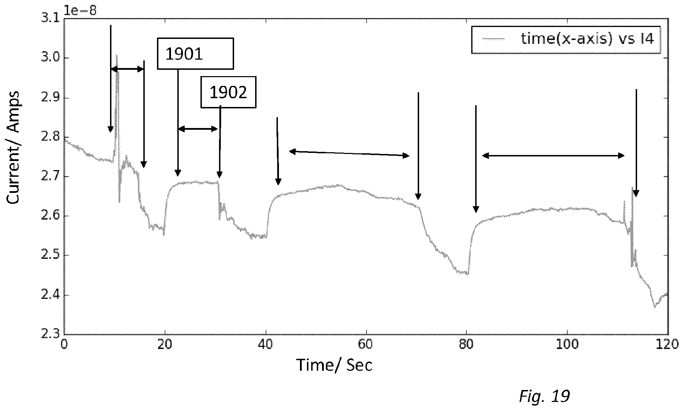

Figure 19 illustrates another graph showing the detection of breath using nanostructure array composed of carbon nanotubes (CNTS) as field emission based sensing devise. A voltage is applied between two adjacent nanostructures and the current (field emission current) is measured. The arrows (1901) indicate the time when the CNT nanostructures are exposed to human breath and the arrow (1902) indicates the time when the exposure to human breath was removed. It can be see that the field emission current (y axis) between the nanostructures increases when the nanostructures are exposed to human breath. Hence, gases can be detected using this technique.

## Claims

1. An arrangement of at least two individually addressable nanostructures (207) in an array on a substrate (201),
wherein the substrate (201) is non-conducting, wherein there are conducting electrical portions (208) within the substrate,
wherein the conducting electrical portions form electrical contacts with the nanostructures (207) forming the individually addressable nanostructures in an array,
wherein the nanostructures (207) are individually connected with conductive paths (403) on the first face (202) of the non-conducting substrate (201) and conductive structures (210) in a second substrate (209) via the conductive portion (208) in the first substrate (201) wherein the said nanostructures (207) are covered with a medium (3000), and
wherein when a voltage (900) is applied between the at least two nanostructures (207), an electric or electromagnetic field is generated between the said nanostructures and a capacitance (700) is formed between the nanostructures;
optionally wherein the nanostructures (207) are selected from the group consisting of: nanotubes, nanofibers, nano rods and nano wires;
optionally wherein the nanostructures (207) are selected from carbon nanotubes, carbon nanofiber, silicon nanowires, zinc oxide Nano rods.

2. The arrangement of claim 1, wherein the electrical field results in movement of charged material (800) between the nanostructures;
optionally wherein each nanostructure (207) has a base size (2210), wherein the base size (2210) ranges from about 1-1 000,000 nm;
optionally wherein the height (2220) ranges from about 10 - 1 000 000 nm;
optionally wherein the distance, (2213) is the gap between each nano-material that ranges from 1-100 nm;
optionally wherein the at least two nanostructures (207) are separated from each other by a distance (800), wherein the distance (800) ranges from 1-100000 nm;
optionally wherein the at least two nanostructures are charged with a positive charge or negative charge by the electrical portion in substrate;
optionally wherein each nanomaterial has a base size (2212) of 1-100 nm and height (2211) that ranges from 1-1 000,000 nm.

3. The arrangement of claim 1 wherein the medium (3000) is a solid surface or a liquid or a gas; optionally wherein the medium is stationary or in a flow;
optionally wherein the medium is selected from the group consisting of vacuum, air, gas mixtures, polymer, ceramics, silicon, semiconductors, metals, silicone, quarts, mica, Teflon, oil, solutions and liquid mixtures;
optionally wherein the medium (3000) is at least about 1- 500000 nm thicker than the height of the nanostructures.

4. The arrangement according to claim 2 wherein the voltage (900) can be applied between the nanostructures and an external electrode; optionally wherein the material of the external electrode can be selected from the group consisting of metals, composite materials, semiconductors, conducting polymers, and silver/silver chloride.

5. The arrangement according to claim 1, wherein capacitance (700) is formed between the nanostructures and the direction of the electrical field is dependent on the polarity of the voltage (900) applied.

6. The arrangement according to claim 1, wherein the medium further comprises an analyte (600) in the medium (3000);
optionally wherein the size of the analyte is from 1 angstrom to 1 mm; preferably from 1 nm to 1000 nm; most preferably from 1 angstrom to 10 nm;
optionally wherein the analyte is selected from the group consisting of ions, cells, nano particles DNA, RNA, bio-molecules, polymers, ceramics, metals, gases, bacteria, viruses, vapors, and toxins;
optionally wherein the analyte is a chemical;
optionally wherein the chemical is detected using electrochemical spectroscopy due to electrochemical changes or impedance changes caused by the analyte in the medium;
optionally wherein the analyte is a chemical that can be detected using capacitance changes due to dielectric constant changes in the medium caused by the analyte;
optionally wherein the analytes is a chemical that can be detected using field emission sensing as the analyte is ionized by the field emission causing a change in the properties of the medium.

7. A device (300) comprising at least two individually addressable nanostructures (207) in an array on a substrate (201),
wherein the substrate (201) is non-conducting with conducing electrical portions (208) within the substrate,
wherein the conducting electrical portions form electrical contacts with the nanostructures (207) forming the individually addressable nanostructures in an array,
wherein the nanostructures (207) are individually connected with conductive paths (403) on the first face (202) of the non-conducting substrate (201) and conductive structures (210) in a second substrate (209) via the conductive portion (208) in the first substrate (201) wherein the said nanostructures (207) are covered with a medium (3000), and
wherein when a voltage (900) is applied between at least two nanostructures (207), an electric or electromagnetic field is generated between the said nanostructures and a capacitance (700) is formed between the nanostructures;
optionally wherein the electrical field results in movement of charged material (800) between the nanostructures.

8. The device according to claim 7, wherein at least one nanostructures (207) in the array can be charged with a first charge and at least a second nanostructures (207) in the array can be charged with a second charge;
optionally wherein the electrical interaction between the first set and the second set of nanostructures will generate a first electrical signal,
wherein external perturbation or presence of analyte (600) in the medium (3000) creates a change the electric field;
optionally wherein the electrical interaction between the first set and the second set of nanostructures will generate a first electrical signal,
wherein external perturbation or presence of analyte (600) in the medium (3000) creates a change the capacitance (700);
optionally wherein the electrical interaction between the first set and the second set of nanostructures will generate a first electrical signal,
wherein external perturbation or presence of analyte (600) in the medium (3000) creates a change the flow of charged materials between the said two nanostructures causing a change that can generate a second electrical signal.

9. The device according to claim 7, for use as an electrochemical, capacitive and/or field emission sensor array;
optionally wherein the nanostructures act as a nano electrode array for electrochemical detection of analytes (600) in the medium (3000),
wherein the arrangement is employed is as capacitive sensing device wherein the nanostructures act as nano electrode array for capacitive sensing of analytes (600) in the medium (3000), and
wherein the arrangement is employed as a field emission based sensing device wherein the nanostructures act as nano electrode array for field emission based sensing of analytes (600) in the medium (3000); optionally wherein the nanostructures are functionalized.

10. The device according to claim 9, wherein the functionalization is performed via covalent functionalization, surface adsorption, electro-polymerization or electrochemical deposition;
optionally wherein the functionalization of the nanostructures enhances the charging on the nanostructures;
optionally wherein the functionalization of the nanostructures enhances the sensing of multianalyte simultaneously.

11. The device according to claim 7, wherein the said conductive portion in the insulating layer is photovoltaic (208);
optionally wherein the said conducting portion (208) produces electricity when the material is exposed to light;
optionally wherein exposure to light allows energy harvesting from electromagnetic waves to electricity for self-powering device.

12. A system (4000) comprising the device (300) of claim 7 and a chip holder (4401); optionally wherein the chipholder (4401) provides at least one electrical contact with the nanostructure array device (300);
optionally wherein the chipholder (4401) provides microfluidics for medium (3000) around the nanostructure arrays (207);
optionally wherein the chipholder (4401) provides an electrical connection for external hardware (4402);
optionally wherein he external hardware (4402) comprises data acquisition and signal generation hardware electronics;
optionally wherein the hardware (4402) is connected to a software (4403) using a wired or wireless connection, and
wherein the software (4403) process the data generated from the device (300).

13. A method of monitoring, detecting or manipulating of cells using the system of claim 12;
optionally wherein manipulation of cell includes cell poration,
wherein an electric charge is delivered to the cell membrane (1401) using the nanostructure (207),
wherein the electric charge causes a shock to the cell, and
wherein the cell membrane open up (1404) at site-specifically at the nanostructure (207) used to deliver the electric charge;
optionally wherein one or more chemicals and/or analytes in a cell and around a cell can be detected,
wherein the detection of chemicals and analytes include the intra cell analyte measurements, measurements of potentials and analytes across cell membrane, analyte measurement in the micro environment of the cells using electrochemical, capacitive and field emission techniques;
optionally wherein cell monitoring includes a cell that is monitored for movement, chemical and analyte excretion and intake using electrochemical, capacitive or field emission sensing, wherein the cell is a single cell in isolation in a medium (3000),
wherein the cell is a single cell in a population of cells in a medium, or
wherein the cell is in interaction with multiple other cells;
optionally wherein the detection cell includes detection of chemicals and analytes, wherein the chemicals and analytes are in the micro environment of single cell in the medium (3000),
wherein the chemical activity of the single cell membrane can be detected with special resolution using individually addressable nanostructures, and wherein the cells are in vivo or in vitro.

14. A method of multianalyte detection using the system of claim 12;
optionally wherein detection is carried out using one or more of electrochemical spectroscopy, capacitive sensing or field emission sensing.
